# EUROPEAN PATENT APPLICATION

(11) **EP 4 468 310 A1**
(43) Date of publication of application: **27.11.2024**
(21) Application number: 23178328.3
(22) Date of filing: 09.06.2023
(51) Int. Cl.: G16H 50/20

(54) **ADAPTIVE DECISION-SUPPORT THROUGH CONNECTION OF HIERARCHAL DATA MODELS**

(30) Priority: 26.05.2023 US 202363469083 P
(71) Applicant: Koninklijke Philips N.V., 5656 AG Eindhoven (NL)
(72) Inventor: JACOBS, Igor, Eindhoven (NL); VERBURG, Pepijn, Eindhoven (NL); VAN DE PUT, Timo, 5656AG Eindhoven (NL); PLUYTER, Jon Ragnar, Eindhoven (NL); LOOS, Marc, Eindhoven (NL); VAN OMMERING, Rob, Eindhoven (NL)
(74) Representative: Philips Intellectual Property & Standards

(57) **Abstract**

The present invention relates to the field of clinical decision support. In order to provide an improved approach for generating subject-specific user interfaces for clinical decision support, there is provided a method for generating an adaptive dashboard for clinical decision support, the method comprising:
a) selecting (210) a knowledge tree constructed according to a set of clinical knowledge data associated with a defined disease condition and/or a defined subject context, wherein the knowledge tree comprises a plurality of interrelated nodes representing clinical information categories, the nodes having a respectively associated pruning condition;
b) determining (240) availability of the clinical information categories of the selected knowledge tree in a patient record and relevance of the clinical information categories of the selected knowledge tree to the patient context and/or the disease condition;
c) pruning (230) at least one of the nodes on the selected knowledge tree based on at least one of the pruning conditions being satisfied by the determined information availability and the determined information relevance, resulting in a pruned knowledge tree with a subgroup of clinical information categories; and
d) generating (240) the adaptive dashboard by mapping the subgroup of clinical information categories to a dashboard template.

## Description

### FIELD OF THE INVENTION

The present invention relates to the field of clinical decision support, and more particularly to a method and a data processing apparatus for generating an adaptive dashboard for clinical decision support, and to a clinical decision support system.

### BACKGROUND OF THE INVENTION

In healthcare, there is a trend towards increased precision in how diagnostic information is acquired and shared. Diagnostic assessment and treatment evaluation is not only performed by radiologic imaging and pathologic evaluation alone anymore, but disease characterization at the genetic level has become increasingly important. For each cancer patient, there will be multiple moments along the care path at which clinicians need to carefully review this information to decide on the disease state and next step.

With the transformation of healthcare towards precision diagnosis and personalized treatment selection, the clinical decision-making process has become increasingly complex. There is a need for solutions that provide clinicians with access to contextually relevant information at each of such key decision moments.

Clinical decision-support systems (CDSS) are designed to aid clinicians in decision-making and improve patient outcomes through providing an actionable overview of patient information and aggregated diagnostic and treatment data. Such data may be presented along with knowledge bases or other sources of information tailored to the patient's profile. It is important that CDS dashboards can provide a longitudinal overview, as patients often go through multiple cycles of diagnosis and treatment and frequently have multiple medical conditions. Such overview would present the relevant current disease state along with contextually relevant disease and treatment history.

Clinical data presentation could be driven by diagnostic and treatment pathways. Such pathways define the information required at each decision moment and how they impact next-step decisions. For example, US 2022/0215961 A1 describes a method for generating subject-specific user interfaces for clinical decision support. This method generates user-interface components and/or elements for a subset of subject-specific clinical information, based on the available subject-specific clinical information, clinical context, and clinical reference information from established clinical knowledge. These user elements or components could present themselves in various formats (e.g. small/medium/large) in a way that the available screen real estate was effectively used.

However, many decision-support dashboards available today cannot flexibly display subsets of information in a hierarchal way, tailored to support specific clinical questions for the patient at hand at each defining moment along the care path. They often display either too generic information that does not aid the decision-making, or display too much information at once in a too granular way. This hampers their clinical value and adoption in the field.

What is needed is decision-support that can zoom into particular clinical questions, e.g. assessment of the primary diagnosis, re-staging after disease recurrence, treatment response evaluation after various lines of treatment, or assessment of the impact of a comorbid condition on suitability of a particular treatment.

The medical information presented in relation to these questions typically comes from a variety of data sources, including radiologic imaging, pathology, genetic testing, and other patient information from electronic health records. The ability to capture such information in a structured or standardized way is likely to increase with the advent of (artificial intelligence) algorithm-based image and report processing techniques and structured reporting initiatives. The patient information could then be aggregated in patient data models that describe the hierarchal relation among data elements captured along the care path.

### SUMMARY OF THE INVENTION

There may be a need to provide an improved approach for generating subject-specific user interfaces for clinical decision support.

The present invention is defined by the independent claims, wherein further embodiments are incorporated in the dependent claims. It should be noted that the following described aspects of the invention apply also for the method, the data processing apparatus, and the clinical decision support system. A subject may, e.g., be a patient.

According to a first aspect of the present invention, there is provided a method for generating an adaptive dashboard for clinical decision support, the method comprising:
a) selecting a knowledge tree constructed according to a set of clinical knowledge data associated with a defined disease condition and/or a defined subject context, wherein the knowledge tree comprises a plurality of interrelated nodes representing clinical information categories, the nodes having a respectively associated pruning conditions;
b) determining availability of the clinical information categories of the selected knowledge tree in a subject record and relevance of the clinical information categories of the selected knowledge tree to the subject context and/or the disease condition;
c) pruning at least one of the nodes on the selected knowledge tree based on at least one of the pruning conditions being satisfied by the determined information availability and the determined information relevance, resulting in a pruned knowledge tree with a subgroup of clinical information categories; and
d) generating the adaptive dashboard by mapping the subgroup of clinical information categories to a dashboard template.

According to a second aspect of the present invention, there is provided a data processing apparatus for generating an adaptive dashboard for clinical decision support, the layout engine comprising a processing unit configured to perform the method according to the first aspect and any associated example.

According to a third aspect of the present invention, there is provided a clinical decision support system, comprising:
- a dashboard template database (20) configured to store a plurality of dashboard templates, each dashboard template having a hierarchal composition of dashboard layout elements usable for presenting clinical information from one or more clinical information categories;
- a knowledge tree database (30) configured to store a plurality of knowledge trees stored in a knowledge tree database, each stored knowledge tree being constructed according to a set of clinical knowledge data associated with a defined disease condition and/or a defined subject context, wherein each knowledge tree comprises a plurality of interrelated nodes representing clinical information categories to be presented, the nodes having a respectively associated pruning condition;
- a subject case management system (40) configured to store subject records of one or more subjects; and
- a data processing apparatus (10) according to claim 13 configured to receive a dashboard request and to generate an adaptive dashboard for clinical decision support based on the received dashboard request.

Optionally, the clinical decision support system may further comprise a clinical guidelines and pathways database configured to store clinical guidelines and pathways.

Accordingly, the present disclosure describes a method, an apparatus, and a clinical decision support system to generate adaptive user interfaces for clinical decision support. The method, the apparatus, and the clinical decision support system may utilize a dashboard template that serves as a basis for adaptive layouts. These dashboard template may comprise both static layout areas, as well as areas of which the content may adaptively represent different data at various decision moments and in various contexts. For configuration of the layouts, the method, the apparatus, and the clinical decision support system may utilize knowledge tree structures to represent clinical information categories. The clinical information categories are represented by user interface components associated with the knowledge tree structure. The method, the apparatus, and the clinical decision support system may apply knowledge tree filters to prune the knowledge trees and hence affect which knowledge categories are displayed on the dashboard at various decision moments. This may depend on availability and relative priority of data available in the subject database. Besides, the relative priority may be influenced by knowledge bases such as clinical guidelines or clinical pathways that define required data for decision-making at various moments along the subject's care path. The translation from a knowledge tree to the screen-based user interface is managed by the layout engine, which maps the knowledge tree to possible layout solutions and derives the optimal layout by calculating scores for intermediate layouts and iteratively optimizing screen usage by permutating the layout. The proposed approach may filter and prioritize the relevant information and effectively communicate these through a dedicated user interface. The generated decision-support dashboards may thus flexibly display subsets of information in a hierarchal way, tailored to support specific clinical questions for the subject at hand at each defining moment along the care path.

In other words, the present disclosure provides a different approach for generating subject-specific user interfaces for clinical decision support. In this proposed approach, the starting point for generating a personalized user interface is a knowledge tree that describes a subject's disease state and history. It may have different states at various time points along the care path. In a next step, the knowledge tree is pruned or filtered e.g., based on a disease condition and/or a subject context, which may be dependent on the subject's position in the care path and context. In a final step, the knowledge tree is mapped to possible layout solutions, based on which a desired layout solution is calculated.

Optionally, the proposed approach may prioritize the relevant information and effectively communicate these through a dedicated user interface. This priority may be proportional to the proposed allocated screen space to each knowledge category. In this way, user interface (UI) components with higher relative importance can be given more space to show more detailed data.

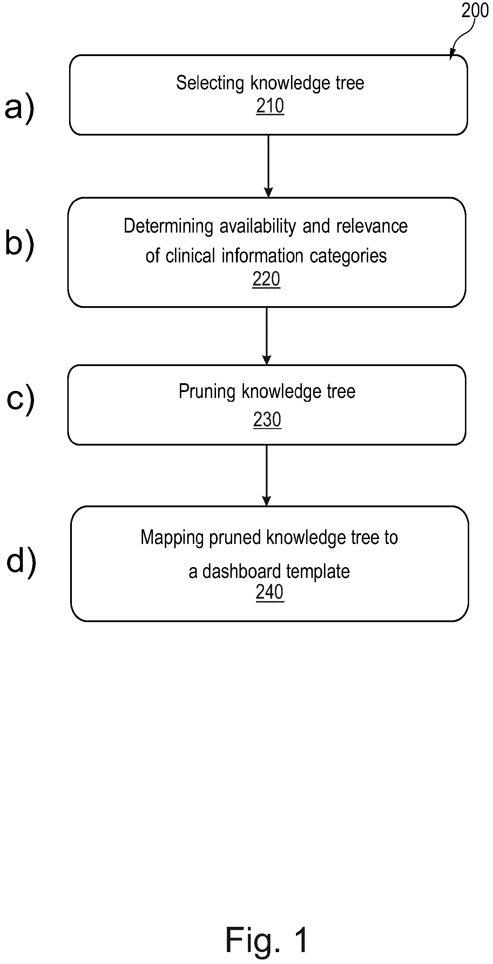

The method will be explained in detail hereinafter and in particular with respect to the example shown in Fig. 1.

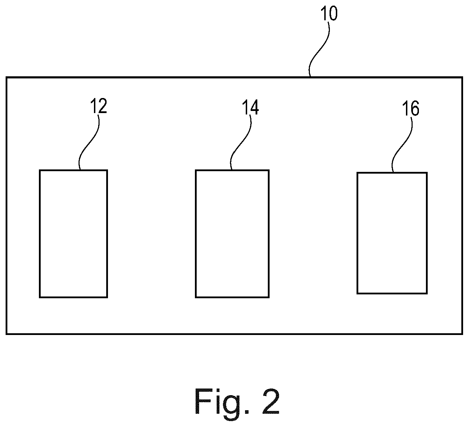

The apparatus will be explained in detail hereinafter and in particular with respect to the example shown in Fig. 2.

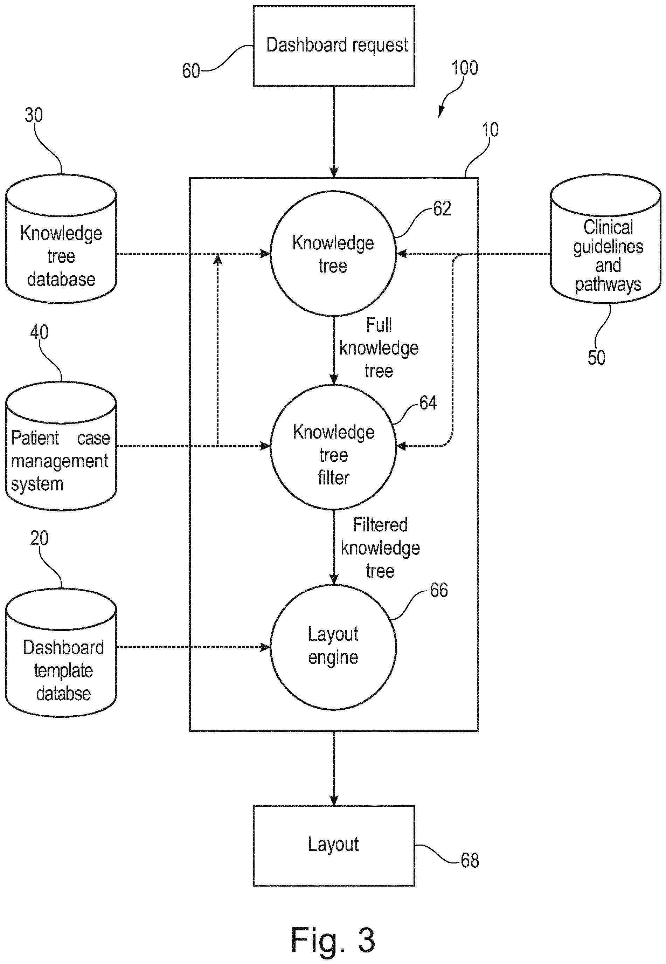

The clinical decision support system will be explained in detail hereinafter and in particular with respect to the example shown in Fig.3.

According to an invention, the method further comprises:
- receiving a dashboard request; and
- selecting the dashboard template from a plurality of dashboard templates according to the received dashboard request.

The dashboard request may comprise information used to indicate one or more of the following of a subject: a disease condition, subject identifier information, and a subject context.

The plurality of dashboard templates may be retrieved from a dashboard template database. Each stored dashboard template may comprise a hierarchal composition of dashboard layout elements usable for presenting clinical information from one or more clinical information categories.

According to an embodiment of the present invention, the step of selecting the dashboard template further comprises:
- assigning a matching score to each of the plurality of dashboard templates, said matching score indicative of a relevance of the associated template information of the respective dashboard template to the dashboard request;
- ranking the dashboard templates according to the associated matching scores; and
- selecting the dashboard template from the plurality of dashboard templates based on the ranking.

In other words, the selection of an appropriate dashboard template may be based on a ranking system. For example, the ranking system may be applied to layouts available in the database, specifying that e.g., disease-specific layouts or customer-specific configurations of layouts overrule default layouts. This may be achieved by a scoring mechanism that uses the "type" of the layout, "mandatory" keys and "preferred" keys of the available layouts. Each of the keys in the layout refer to a list of values as potential matches the context provided in the layout request. For example, each dashboard template stored in the knowledge tree database may have associated template information that may comprise one or more of: an associated type, an associated mandatory key, and a preferred key. This will be explained in detail hereinafter and in particular with respect to the exemplary method shown in Fig. 1.

According to an embodiment of the present invention, the nodes further comprise respectively associated priorities, which are usable to determine respectively associated areas in the adaptive dashboard occupied by the subgroup of clinical information categories.

For example, each clinical information category of the tree may be assigned a UI component with higher relative importance, e.g., higher priority, may be given more space to show more detailed data.

According to an embodiment of the present invention, at least one of the respectively associated priorities is adjustable at different decision moments along a subject's clinical pathway.

For example, the associated priorities of the remaining clinical information categories of the pruned knowledge tree may be adjusted based on the determined relevance to the subject context, e.g., based on a knowledge base that defines required data for decision-making at different decision moments along a subject's clinical pathway.

According to an embodiment of the present invention, step d) further comprises iteratively mapping the subgroup of clinical information categories to dashboard layout elements of the selected dashboard template including resolving spatial constraints based on the priorities associated with the clinical information categories in the subgroup.

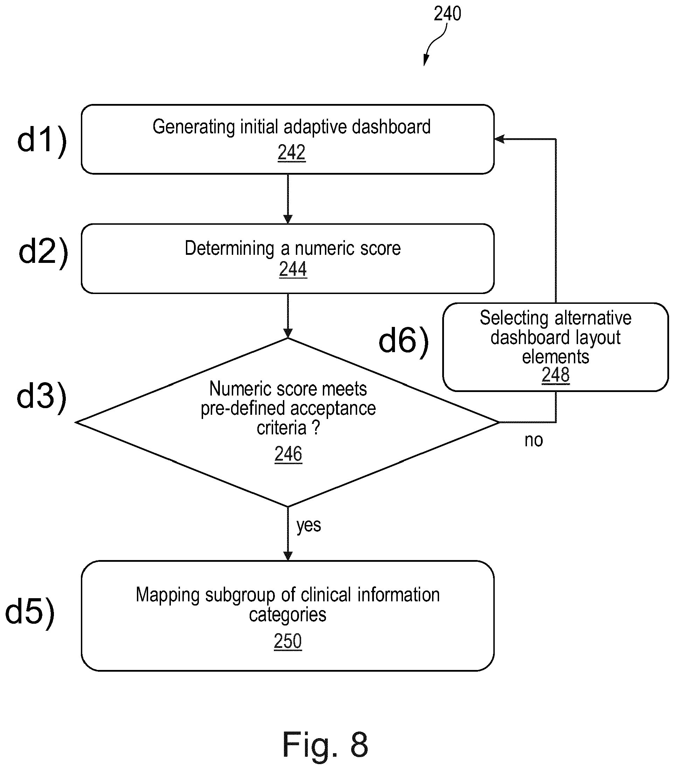

The translation from a knowledge tree to the screen-based user interface may be managed by the layout engine, which maps the knowledge tree to possible layout solutions and derives the optimal layout by calculating scores for intermediate layouts and iteratively optimizing screen usage by permutating the layout. This will be explained in detail hereinafter and in particular with respect to the example shown in Fig. 8.

According to an embodiment of the present invention, the step of iteratively mapping the subgroup of clinical information categories to the dashboard layout elements of the selected dashboard template comprises:
d1) generating an initial adaptive dashboard by mapping the subgroup of clinical information categories to the dashboard layout elements of the selected dashboard template;
d2) determining a numeric score of the initial adaptive dashboard based on one or more of:
   (i) a degree of an occupied area matching the associated priority of the represented clinical information category;
   (ii) a proportion of empty space in the generated adaptive dashboard; and
   (iii) a degree of a total dashboard area size fitting an available screen area;
d3) determining whether the determined numeric score meets pre-defined acceptance criteria;
d4) in response to determining that the determined numeric score does not meet the pre-defined acceptance criteria, updating the selected dashboard template by selecting alternative dashboard layout elements representing the subgroup of clinical information categories and/or changing an layout direction of the selected dashboard template, and repeating steps d1)-d3) until the determined numeric score meets the pre-defined acceptance criteria; and
d5) in response to determining that the numeric score meets a pre-defined acceptance criteria, mapping the subgroup of clinical information categories to the dashboard layout elements of the updated dashboard template.

This will be explained in detail hereinafter and in particular with respect to the example shown in Fig. 8.

According to an embodiment of the present invention, the clinical information categories comprise one or more of:
- subject data describing at least one physiological parameter or characteristic of the subject;
- clinical data describing at least one medical parameter or characteristic of the subject; and
- operational data describing at least one clinical test or procedure undertaken by the subject; and
- result data describing at least one result or outcome from diagnostic tests and treatment procedures.

According to an embodiment of the present invention, the information in the dashboard request comprises one or more of:
- a disease code that specifies one disease condition or a combination of disease conditions of the subject;
- subject identifier information of the subject usable to retrieve subject-specific information, from which the disease condition and/or the subject context are derivable; and
- subject context information that specifies the subject context.

According to an embodiment of the present invention, the subject context comprises one or more of:
- a position of the subject in a subject's clinical pathway;
- a clinical discipline triggering the dashboard request; and
- a context of use of the adaptive dashboard.

According to an embodiment of the invention, at least one dashboard template comprises one or more adaptive elements usable for adaptively representing different data for different subject contexts and/or different disease conditions.

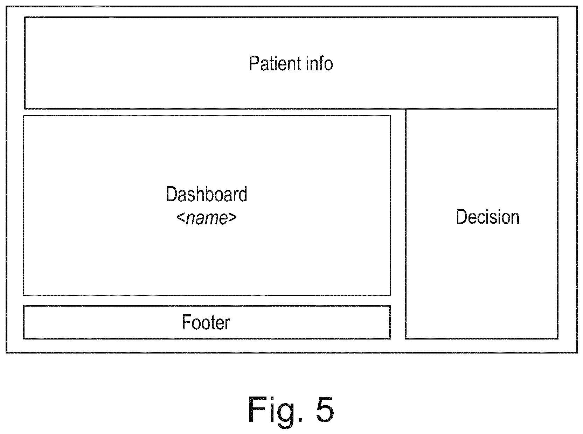

An exemplary dashboard template with adaptive elements is shown in Fig. 5.

According to an embodiment of the present invention, at least one dashboard template is a disease-specific dashboard templates being constructed for a particular disease condition.

In other words, the dashboard templates may include templates that may be preconfigured for various disease conditions, e.g., lung cancer, prostate cancer, cardiovascular disease, etc. Each dashboard template has a hierarchal composition of dashboard layout elements usable for presenting clinical information from one or more clinical information categories.

According to an embodiment of the present invention, the knowledge tree database comprises:
- at least one knowledge tree being constructed by a recursive tree of clinical information categories; and/or
- at least one knowledge tree being constructed by a graph-like structure.

According to examples, there is provided a computer program product comprising instructions which, when the program is executed by a processing unit, cause the processing unit to carry out the steps of the method according to the first aspect and any associated example.

According to examples, there is provided a computer-readable storage medium having stored thereon the computer program product.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 illustrates a flowchart describing a method for generating an adaptive dashboard for clinical decision support.
Fig. 2 illustrates an exemplary data processing apparatus.
Fig. 3 illustrates an exemplary clinical decision support system.
Fig. 4A shows a conceptual illustration of a knowledge tree.
Fig. 4B shows a conceptual illustration of a pruned knowledge tree.
Fig. 5 illustrate an exemplary dashboard template which may comprise both fixed and adaptive parts.
Fig. 6A illustrates an exemplary pruned knowledge tree.
Fig. 6B illustrates a dashboard that comprises hierarchal composition of a layout that can be represented by the knowledge tree shown in Fig. 6A.
Figs. 7A-7C shown different component variants to represent various levels of details about the same medical aspect.
Fig. 8 illustrates a flowchart describing a method for iteratively mapping the subgroup of clinical information categories to the dashboard layout elements of the selected dashboard template.
Fig. 9 illustrates how the remaining space can be filled by growing the selected components within the dimensions of the enclosing area.

### DETAILED DESCRIPTION OF EMBODIMENTS

Adoption and effective use of clinical decision support systems is limited, since they often fail to filter and prioritize the relevant information and effectively communicate these through a dedicated user interface.

Due to the amount and complexity of data relevant for an individual subject (e.g., a patient) at various decision moments, rigid user interfaces based on general templates are unable to effectively support decision making. For example, data dense templates can cause information overload whereas generalized templates may omit key information. Development of custom-made designs to support all decision points in the diverse care paths of a wide-variety of subjects is practically infeasible due to the required development time and cost. Similarly, when the end-user is enabled to configure the user interface, not only may the end-user have insufficient insight into all relevant multidisciplinary subject information at the various decision moments, it is also practically infeasible to make a configuration for each specific subject and each decision moment related to that specific subject.

To this end, the present disclosure provides a data processing method and apparatus for generating an adaptive dashboard for clinical decision support and a clinical decision support system. In some implementations, the starting point for generating a personalized user interface is a data model or knowledge that describes a subject's disease state and history. It may have different states at various time points along the care path. In a next step, the data model or knowledge tree is pruned or filtered based on the subject's position in the care path and/or context. In a final step, the data model or knowledge tree is mapped to possible layout solutions, based on which the optimal layout solution is calculated.

Fig. 1 illustrates a flowchart describing an exemplary method 200 for generating an adaptive dashboard for clinical decision support. The illustrated flowchart describes the pipeline to process a dashboard request from a client to a layout that can be rendered on a screen-based interface. The method 200 may be a computer-implemented method, which may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages.

As an example, the exemplary method 200 shown in Fig. 1 may be implemented by a data processing apparatus. Fig. 2 illustrates an exemplary data processing apparatus 10. In the illustrated example, the exemplary data processing apparatus 10 comprises an input unit 12, a processing unit 14, and an output unit 16.

The input unit 12 may include hardware and/or software to enable the data processing apparatus 10 to receive a dashboard request, and the output unit 16 may comprise hardware and/or software to enable to data processing apparatus 10 to communicate with other devices (e.g., viewing platform like monitor, computer screen, tablet screen, etc.) and/or a network (e.g., Internet). As an example, the input unit 12 may comprise or be coupled to an input device, such as a mouse, keyboard, scanner, microphone, camera, etc. In some examples, the input unit 12 and the output unit 16 may include or to be coupled to, a NIC or modulator/demodulator (for accessing remote devices, other files, devices, systems, or a network), a radio frequency (RF) or other transceiver, a telephonic interface, a bridge, a router, etc.

In general, the exemplary data processing apparatus 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 2 may show a limited number of components by way of example, it can be appreciated that a greater or a fewer number of components may be employed for a given implementation.

In some implementations, the exemplary data processing apparatus 10 may be embodied as, or in, a device, such as a server, workstation, or mobile device. The data processing apparatus 10 may comprise one or more microprocessors or computer processors, which execute appropriate software. The processing unit 14 of the exemplary data processing apparatus 10 may be embodied by one or more of these processors. The software may have been downloaded and/or stored in a corresponding memory, e.g., a volatile memory such as RAM or a non-volatile memory such as flash. The software may comprise instructions configuring the one or more processors to perform the functions as described herein.

It is noted that the data processing apparatus 10 may be implemented with or without employing a processor, and also may be implemented as a combination of dedicated hardware to perform some functions and a processor (e.g., one or more programmed microprocessors and associated circuitry) to perform other functions. For example, the functional units of the data processing apparatus 10, e.g., the input unit 12, the processing unit 14, and the output unit 16, may be implemented in the device in the form of programmable logic, e.g., as a Field-Programmable Gate Array (FPGA). In general, each functional unit of the exemplary data processing apparatus 10 may be implemented in the form of a circuit.

In some implementations, the exemplary data processing apparatus 10 may also be implemented in a distributed manner. For example, some or all units of the data processing apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

In some implementations, the exemplary data processing apparatus 10 may be implemented in a cloud-computing environment, which is a paradigm that moves resources, services and applications into a cloud enabling users to access and utilise the resources, services and applications.

The data processing apparatus 10 may be implemented in an exemplary clinical decision support system shown in Fig. 3. The exemplary clinical decision support system 100 shown in Fig. 3 comprises a data processing apparatus 10, a dashboard template database 20, a knowledge tree database 30, and a subject case management system 40. Optionally, as shown in Fig. 3, the exemplary clinical decision support system 100 may further comprise a clinical guidelines and pathways database 50.

The data processing apparatus 10 may be connected to the dashboard template database 20, the knowledge tree database 30, the subject case management system 40, and the clinical guidelines and pathways database 50 via a wired (e.g., cable) or wireless connection (e.g., WLAN). Although Fig. 3 may show separate devices by way of example, it can be appreciated that some devices shown in Fig. 3 may be implemented in a single device. For example, the knowledge tree database may be stored in the data processing apparatus 10. In some implementations, the exemplary clinical decision support system 100 may be implemented in a distributed manner. For example, some or all devices of the clinical decision support system 100 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

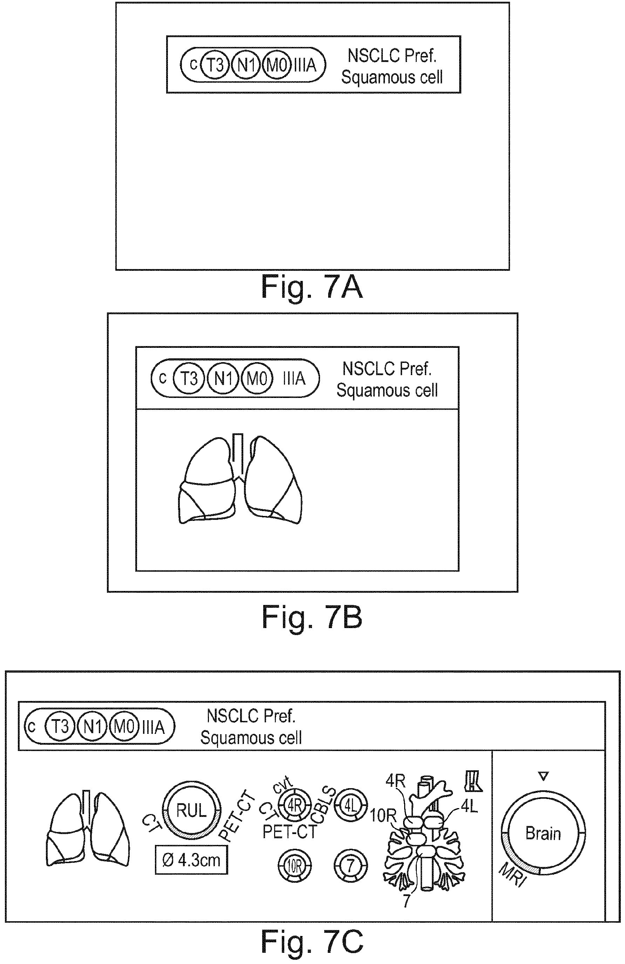

The dashboard template database 20 may also be referred to as layout template database. The dashboard template database 20 is configured to store a plurality of dashboard templates. Each dashboard template may comprise a plurality of user-interface (UI) components or elements. Some UI elements/components may be available in multiple configurations or appearances and with various degrees of detail and emphasis including, but not limited to, different sizes, different graphical representations for e.g., summarized and extended views, different highlighting methods, to e.g., stress novelty of data or discrepancy in data. Exemplary multiple alternative representations for the same UI component are shown in Figs. 7A-7C. The plurality of dashboard templates may include generic templates and/or standard templates. As an example, a generic template may contain a dashboard header with empty content area, a subject summary dashboard, and a tumor board dashboard. The standard templates may be preconfigured for various disease conditions, e.g., lung cancer, prostate cancer, cardiovascular disease, etc. Each dashboard template has a hierarchal composition of dashboard layout elements usable for presenting clinical information from one or more clinical information categories. The dashboard templates may serve as a basis for adaptive layouts, where the entire dashboard template or parts of the template may represent adaptive content. An exemplary generic dashboard template is shown in Fig. 5.

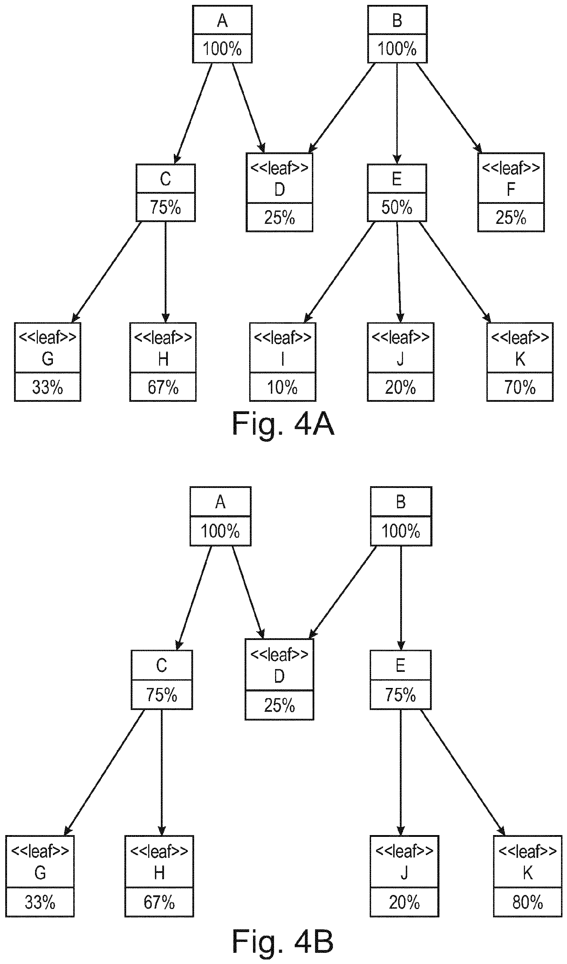

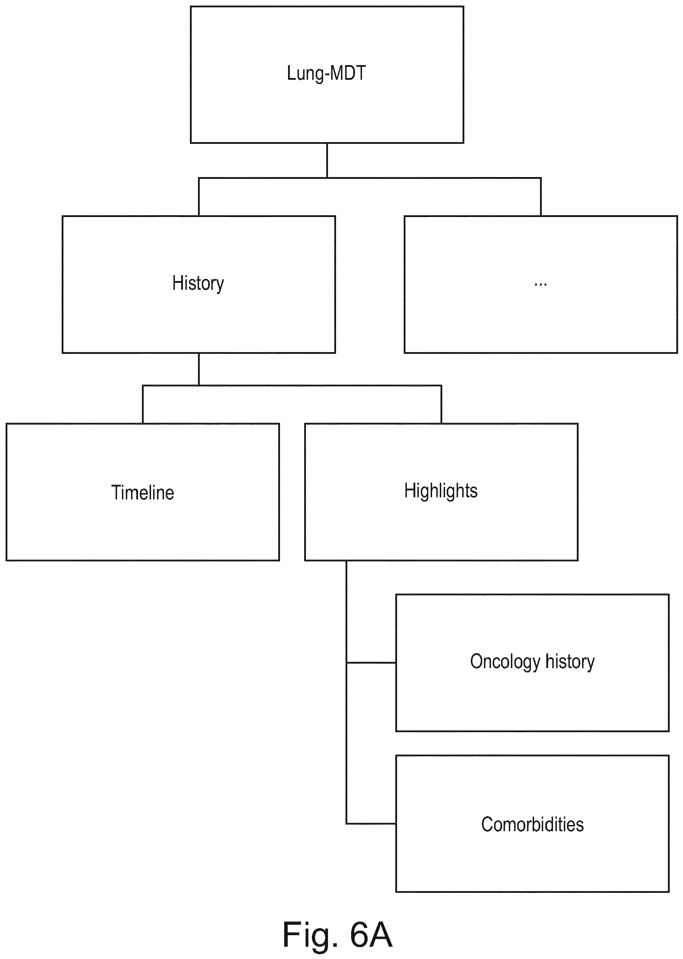

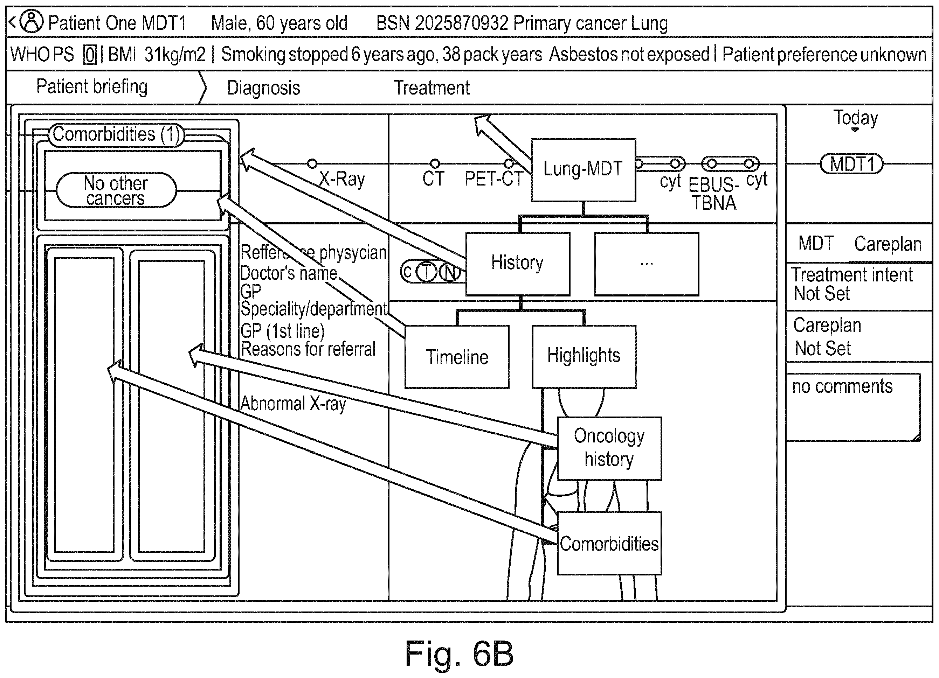

The knowledge tree database 30 is configured to store a plurality of knowledge trees. Each stored knowledge tree is constructed according to a set of clinical knowledge data associated with a defined disease condition and/or a defined subject context. Each knowledge tree comprises a plurality of interrelated nodes representing clinical information categories to be presented. In other words, each knowledge tree contains a set of hierarchal knowledge tree structures, e.g., for a variety of disease conditions, which provide a representation of pieces of knowledge and how they are associated with each other. It also provides the relevant attributes for each category of knowledge inside the tree. Each node and/or leaf has an associated pruning condition and an associated priority. A leaf is a node that does not have additional children nodes. The knowledge trees will be discussed below and in particular with respect to the examples shown in Figs. 4A, 4B, 6A, and 6B.

The subject case management system 40 is configured to store subject records of one or more subjects. The subject case management system 40 may include a database, an application, a local file, or any combination thereof. The subject records may comprise structured data elements according to a subject data model or unstructured data that are merely coded to a specific data type, e.g., unstructured radiology data. The structured data is organized, coded, and may have known relations with other data elements. This may enable that the structured data can for example be populated into dedicated fields of the user interface. Furthermore, relations and associations between structured data elements can be shown. In addition, graphical representations can be used for structured data elements or relations between them to make information more glanceable. Exemplary data records of a subject may comprise one or more of the following subject-specific information: (i) subject data describing at least one physiological parameter or characteristic of the subject, such as demographics, socio-economic, medical history, etc.; (ii) clinical data describing at least one medical parameter or characteristic of the subject, such as subject fitness, complaints, laboratory data, diagnostic data, treatment data, etc.; (iii) operation data describing at least one clinical test or procedure undertaken by the subject, such as date of exams, duration between exams, image, and test quality, etc.; and (iv) result data describing at least one result or outcome from diagnostic tests and treatment procedures.

The clinical guidelines and pathways database 50 may be configured to store clinical guidelines and pathways. Clinical guidelines may comprise a set of recommendations for optimal management of subjects with specific conditions, based on research evidence and clinical practice experience. A subject's care pathway may describe the sequential steps in a subject's multidisciplinary care process. The clinical guidelines and pathways may define decision points, the required information to make the decision, principles and guidelines for evaluation, etc.

In operation, the data processing apparatus 10 may receive a dashboard request 60 e.g., from an input device, such as keyboard, scanner, microphone, etc. The dashboard request 60 comprises information used to indicate one or more of the following of a subject: a disease condition, subject identifier information, and a subject context. For example, the disease condition may be represented by a disease code that specifies one disease condition or a combination of different disease conditions of the subject. The disease condition may be specified as the relevant disease condition in a particular context, or may be derived from the subject-specific information. The subject identifier information may comprise a reference number to an individual subject and may be used to retrieve subject-specific information. The subject context information may comprise one or more of the position of the subject in the care pathway, the clinical discipline triggering the dashboard request, the context of use of the dashboard, and other contextual information.

After receiving the dashboard request 60, the data processing apparatus 10 may access the knowledge tree database 30 to select a knowledge tree 62 from a plurality of knowledge trees stored in a knowledge tree database 30. The selected knowledge tree 62 may be constructed according to a set of clinical knowledge data associated with a disease condition and/or a subject context that match the disease condition and/or the subject context in the dashboard request. The selected knowledge tree 62 comprises a plurality of interrelated nodes and leaves representing clinical information categories to be presented by user interface components associated with the knowledge tree structure. Each node and/or leave has an associated pruning condition and an associated priority. As shown in Fig. 3, the selected knowledge tree may also be referred to as full knowledge tree.

The data processing apparatus 10 further applies a knowledge tree filter 64 to prune the selected knowledge tree and hence affect which clinical information categories are displayed on the dashboard e.g., at various decision moments. This may be implemented by the processing unit 14 of the data processing apparatus 10 shown in Fig. 2. This may depend on availability and relative priority of data available in the subject case management system 40. For example, the data processing apparatus 10 may access to the subject case management system 40 to retrieve subject records of the subject. The subject records of the subject may comprise one or more of the following subject-specific information: (i) subject data: demographics, socio-economic, medical history, etc.; (ii) clinical data: subject fitness, complaints, laboratory data, diagnostic data, treatment data, etc.; (iii) operation data: date of exams, duration between exams, image and test quality, etc.; and (iv) result data describing at least one result or outcome from diagnostic tests and treatment procedures. The availability of the subject-specific information may vary between subjects and at various decision moments. For example, the subject records of some subjects may comprise certain operation data, while the subject records of other subjects may not. Even for the same subject, the subject record may change e.g., along the clinical pathway. The data processing apparatus 10 determines availability of the clinical information categories of the selected knowledge tree in the subject record, and prunes the selected knowledge tree based on the clinical information categories available for the subject. Additionally, the data processing apparatus 10 also determines relevance of the clinical information categories of the selected knowledge tree to the subject context and/or the disease condition in the dashboard request, and prunes the selected knowledge tree based thereon. Pruning only applies to a limited set of knowledge categories, as for some categories it is equally valuable to show there is no information for the subject. As shown in Fig. 3, the pruned knowledge tree may also be referred to as filtered knowledge tree. An example of the pruning procedure will be described below and in particular with respect to the examples shown in Figs. 4A and 4B.

The relative priority may be influenced by knowledge bases such as clinical guidelines or clinical pathways that define required data for decision-making at various moments along the subject's care path. In these examples, in order to provide optimal decision-support, clinical data presentation may be driven by diagnostic and treatment pathways. For example, as shown in Fig. 3, the data processing apparatus 10 may further access the optional clinical guidelines and pathways database 50 that comprises information required at each decision moment and how they impact next-step decisions. In some examples, the data processing apparatus 10 may apply the obtained clinical guidelines and pathways to the pruned knowledge tree to adjust the associated priorities of the clinical information categories of the pruned knowledge tree. In some examples, the data processing apparatus 10 may apply the obtained clinical guidelines and pathways to the knowledge tree filter as an additional filtering of data elements at attribute level. This will be explained hereinafter and in particular with respect to the examples shown in Figs. 4A and 4B.

The filtered knowledge tree can be provided to a layout engine 66 of the data processing apparatus 10, which may be implemented by the processing unit 14 of the data processing apparatus 10 shown in Fig. 2. The data processing apparatus 10 may access the dashboard template database 20 to select, based on the dashboard request, a dashboard template from a plurality of dashboard templates stored in a dashboard template database. Each dashboard template has a hierarchal composition of dashboard layout elements usable for presenting clinical information from one or more clinical information categories. An exemplary dashboard template is shown in Fig. 5.

The layout engine 66 may generate the adaptive dashboard layout 68 by mapping the subgroup of clinical information categories to the selected dashboard template. Each clinical information category occupies an associated area in the adaptive dashboard that is dependent on its associated priority. This will be explained hereinafter and in particular with respect to the examples shown in Figs. 6A and 6B.

Turning back to Fig. 1, the method 200 will be described in detail in conjunction with the clinical decision support system 100 shown in Fig. 3.

At block 210, i.e., step a), the method comprises selecting a knowledge tree that is constructed according to a set of clinical knowledge data associated with a defined disease condition and/or a defined patient context. The knowledge tree comprises a plurality of interrelated nodes and leaves representing clinical information categories. The nodes have a respectively associated pruning condition.

The set of clinical knowledge data used for constructing the knowledge trees may be obtained from various sources. In some examples, the set of clinical knowledge data may be obtained from a clinical knowledge base with domain knowledge, rules, and associations. The clinical knowledge base may comprise various forms of clinical reference information including, but not limited to, clinical guidelines or pathways, expert advices, and online data such as research evidence or other literature. Clinical guidelines are a set of recommendations for optimal management of subjects with specific conditions, based on research evidence and clinical practice experience.

The knowledge tree may be selected from a plurality of knowledge trees. In some examples, the plurality of knowledge trees may be provided by a user. In some examples, the plurality of knowledge trees may be stored in a knowledge tree database. The plurality of knowledge trees may be constructed in many ways. In some examples, the plurality of knowledge trees may comprise at least one knowledge tree being constructed by a recursive tree of clinical information categories, where each node contains an ordered list of its children. A knowledge category tree dictionary may be used to maintain a mapping between clinical information category names and the root node for that sub-tree. A lookup in the dictionary may produce a deep copy of the tree that can be pruned without impact on the original tree. Operations like filtering are recursively applied to children. Filtering may be based on conditions that comply with the condition interface. Each condition contains logic that results in numeric scores to base the prioritization of knowledge categories on. In some examples, the plurality of knowledge trees may comprise at least one knowledge tree being constructed by a graph-like structure that is more open for recursion and links between different tree levels.

The condition to prune a leaf from the knowledge tree is also part of the clinical information category definition. Each clinical information category can be annotated with rules that express under which pruning condition the clinical information category should be kept or not kept. If the patient is in the pre-treatment phase, the decisions that need to be made at this moment in time may include: (i) what is the diagnosis and stage, and (ii) what is the recommended treatment? In such case, clinical information categories like an overview of which tests have been performed and an overview of the results of these tests in the patient records of the patient may be relevant information for the diagnosis and stage decision. Relevant information for treatment selection may include the following exemplary clinical information categories: general fitness, organ function, comorbidities, and available treatment options. Therefore, the pruning conditions for the above exemplary clinical information categories may indicate to keep these exemplary clinical information categories if the patient is in the pre-treatment phase. On the other hand, if the patient is in the post-treatment phase, the decisions that need to be made at this moment in time may include: (i) what are the findings at surgery (what is the post-operative stage, what are the treatment margins), and (ii) what is the recommended follow-up (e.g., adjuvant treatment). Therefore, clinical information categories like post-surgical findings, post-surgical stage, resection margins, high-risk factors, may be relevant to the patient context. Therefore, the pruning conditions for the above exemplary clinical information categories may indicate to keep these exemplary clinical information categories if the patient is in the post-treatment phase. The pruning conditions may comprise a simple query to check if a specific field exists (or does not equal null) in the data records of the patient, but it can also check for equality to a specific value, or it can be a combination of multiple conditions.

As an option, the nodes may further comprise respectively associated priorities, which are usable to determine respectively associated areas in the adaptive dashboard occupied by the subgroup of clinical information categories. For example, Fig. 4A shows a conceptual illustration of a knowledge tree of clinical information categories starting from multiple root nodes, such as A and B shown in Fig. 4A. The tree elements are also referred to as "nodes". The lines connecting the tree elements are referred to as "branches". Nodes without children are called "leaf nodes" or "leaves". Each root refers to a sub-tree consisting of nodes and leaves with a priority relative to their parent. The nodes "A" to "K" may represent different clinical information categories, such as medical history, timeline of historical information, highlighted data from that period, categories of information such as oncologic history and other medical history (i.e. comorbidities), and graphical representation of the organ with the abnormality. It will be appreciated that the number and the content of the clinical information categories represented by the nodes and leaves may vary because each knowledge tree is constructed according to a set of clinical knowledge data associated with a defined disease condition and/or a defined patient context.

As shown in Fig. 4A, each clinical information category of the tree is assigned a priority to indicate its relative importance to other categories within the same parent node. In the exemplary knowledge tree show in Fig. 4A, the clinical information category represented by node "A "includes two children, i.e., node "C" and node "D". The clinical information category represented by node "C" is assigned a priority of 75%, and the clinical information category represented by node "D" is assigned a priority of 25%. The difference between the priorities indicates the relative importance of node "C" and node "D" within the same parent node A. This priority is related to the proposed allocated screen space to each clinical information category. For example, the UI components with higher relative importance, i.e., higher priority, can be given more space to show more detailed data.

Turning back to Fig. 1, at block 220, i.e., step b), the method 200 further comprises the step of determining availability of the clinical information categories of the selected knowledge tree in the patient records of the patient. For example, the data processing apparatus 10 shown in Fig. 3 may access the patient case management system 40 to retrieve the patient records of the patient. The records of the patient may comprise one or more of the following patient-specific information: (i) patient data: demographics, socio-economic, medical history, etc.; (ii) clinical data: patient fitness, complaints, laboratory data, diagnostic data, treatment data, etc.; (iii) operation data: date of exams, duration between exams, image and test quality, etc.; and (iv) result data describing at least one result or outcome from diagnostic tests and treatment procedures. If the patient does not have any clinical data available to display for a knowledge category, it may not be worth spending screen real estate to that knowledge category. Hence, knowledge tree filters may be applied that prune these knowledge categories from the tree.

Additionally, at block 220, the method 200 further comprises the step of determining relevance of the clinical information categories of the selected knowledge tree to the patient context and/or the disease condition. As described above, each clinical information category can be annotated with rules that express under which pruning condition the clinical information category should be kept or not kept. In some examples, the clinical information categories may comprise at least one clinical information category with a pruning condition that comprises a simple query to check if a specific field exists (or does not equal null) in the data records of the patient. In some examples, the clinical information categories may comprise at least one clinical information category with a pruning condition that comprises a simple query to check for equality to a specific value. In some examples, the clinical information categories may comprise at least one clinical information category provided with a combination of multiple pruning conditions.

At block 230, i.e., step c), the method 200 further comprises the step of pruning at least one of the nodes on the selected knowledge tree based on at least one of the pruning conditions being satisfied by the determined information availability and the determined information relevance, resulting in a pruned knowledge tree with a subgroup of clinical information categories.

Fig. 4B shows a conceptual illustration of a pruned knowledge tree, which is obtained using pruning to remove nodes from the knowledge tree shown in Fig. 4A. By way of example, the data processing apparatus 10 shown in Fig. 3 may access the patient case management system 40 to retrieve the patient records of the patient and find that the clinical information category represented by node "I" is not present in the patient's records. Consequently, the data processing apparatus 10 may remove the node "I" from the knowledge tree shown in Fig. 4A. Additionally, based on the pruning conditions, the data processing apparatus 10 may further identify that the clinical information category represented by leaf node "F" is irrelevant to e.g., the disease condition and/or patient context in the dashboard request. As a result, the data processing apparatus 10 may further remove the leaf node "F" from the knowledge tree shown in Fig. 4A. After removing the clinical information categories represented by nodes "I" and "F", the priorities of the remaining categories should also be adapted. In the example shown in Fig. 4B, the priority of the clinical information category represented by leaf node "K" may be adjusted to 80%, which is indicative of relative importance to the category represented by leaf node "J" within the same parent node "E". Additionally, the priority of the clinical information category represented by node "E" may be adjusted to 75%, which is indicative of relative importance to the category represented by leaf node "D" within the same parent node "B".

The associated priorities of the remaining clinical information categories of the pruned knowledge tree may be adjusted based on the determined relevance to the patient context, e.g., based on a knowledge base that defines required data for decision-making at different decision moments along a patient's clinical pathway. For example, in the post-treatment phase, the appearance of the post-surgical findings should be the more detailed version, with a focus on post-surgical stage, resection margins, high-risk factors, etc. On the other hand, the appearance of the pre-treatment findings should be the summarized version, only providing the context and highlighting any discrepancies. For this reason, when the patient context indicates that the patient is in the post-treatment phase, the priority of the clinical information categories "post-surgical findings" may be increased, and the priority of the clinical information categories "pre-treatment findings" may be decreased.

At block 240, i.e., step d), the method 200 further comprises generating the adaptive dashboard by mapping the subgroups of clinical information categories to a dashboard template. Each clinical information category may occupy an associated area in the adaptive dashboard that is dependent on its associated priority. In this step, depending on the number of remaining clinical information categories in the pruned tree and their relative priorities, each leaf knowledge category deserves to occupy an area of the layout that is dependent on (e.g., proportional to) its assigned absolute priority. This absolute priority may be calculated recursively for each child by dividing the absolute priority of each parent node across its children according to their relative priority within the parent node.

Fig. 5 illustrates an exemplary dashboard template. As shown in Fig. 5, the exemplary dashboard template may comprise both fixed and adaptive parts. In the illustrated example, the fixed parts comprise patient info, footer, and decision panel, and the adaptive part comprises dashboard <name>. This may enable that generic dashboard components that are not disease-specific are aligned between dashboards. In some other examples (not shown), the dashboard template may only comprise adaptive parts.

In some examples, the dashboard template may be a default dashboard template.

In some examples, the dashboard template may be provided by a user.

In some examples, the dashboard template may be selected based on a dashboard request. In such case, the method 200 may further comprise the steps of receiving a dashboard request, and selecting the dashboard template from a plurality of dashboard templates according to the received dashboard request.

In the step of receiving a dashboard request, the dashboard request may be received e.g., from a user device, which may be a computer, a smartphone, a tablet, a smartwatch, a monitor, or any other device, by which a user can provide the dashboard request via an input device, such as a touch screen, a keyboard, a microphone, a mouse, etc.

In some examples, the dashboard request may comprise information used to indicate a disease condition, e.g., lung cancer, of the patient. The disease condition may comprise a code or a text that specifies the disease condition or combination of disease conditions. This may be specified as the relevant disease condition in a particular context, or may be derived from the patient-specific information.

In some examples, the dashboard request may comprise information used to indicate patient identifier information, which may be a reference number to an individual patient that can be used to retrieve patient-specific information.

In some examples, the dashboard request may comprise information used to indicate a patient context. The patient context may comprise one or more of the position of the patient in the care pathway, the clinical discipline triggering the layout request, the context of use of the dashboard, and/or other contextual information.

In the step of selecting the dashboard template from a plurality of dashboard templates according to the received dashboard request, the dashboard template may be selected from a plurality of dashboard templates stored in a dashboard template database. Each dashboard template may have a hierarchal composition of dashboard layout elements usable for presenting clinical information from one or more clinical information categories. In this step, an appropriate layout is selected based on parameters specified in the dashboard request. This can lead to a ready-to-use (i.e., fixed) layout, an adaptive layout that will run through the pipeline described herein, or may be a composite of a fixed layout with embedded adaptive dashboard components that will run through the pipeline and are after optimization integrated into the overall layout.

The selection of an initial layout may be triggered by the dashboard request, specifying the disease type and/or contextual parameters. The following examples may show how to select different dashboard templates based on the available information in the dashboard request.

In some examples, the dashboard request may comprise information used to indicate a disease condition, e.g., lung cancer, of a patient. In these examples, a generic dashboard template may be selected without knowing the patient context. For example, based on the condition lung cancer, a generic dashboard template may be selected for decision-support for lung cancer patients.

In some examples, the dashboard request may comprise information used to indicate a disease condition and a patient context. In these examples, a version of the lung cancer dashboard template may be generated that focuses on the primary diagnosis. This can be considered a more specialized or focused version of the generic lung cancer dashboard template. Treatment response information may be omitted in this dashboard, since it is known that this does not apply to the patient in this context (i.e. this phase of the care path).

In some examples, the dashboard request may comprise information used to indicate a patient identifier information. From the patient identifier information, the data of the patient may be retrieved, based on which the data to be represented in the dashboard could be identified. In these examples, an even more specialized version of the dashboard template may be selected. For example, in the generic dashboard there was a field to show oncological history. If from the data of the patient with this patient identifier information it is known that the patient doesn't have an oncological history, this information could be omitted from the dashboard.

The selection of an appropriate dashboard template may be based on a ranking system. For example, the ranking system may be applied to layouts available in the database, specifying that e.g., disease-specific layouts or customer-specific configurations of layouts overrule default layouts. This may be achieved by a scoring mechanism that uses the "type" of the layout, "mandatory" keys and "preferred" keys of the available layouts. Each of the keys in the layout refer to a list of values as potential matches the context provided in the layout request.

For example, each dashboard template stored in the knowledge tree database may have associated template information that may comprise one or more of: an associated type, an associated mandatory key, and a preferred key.

The layout type may provide high level information on the context of use. Exemplary layout types may include, but are not limited to, multidisciplinary tumor board, decision-support dashboard, patient consultation dashboard, etc. It will be appreciated that alternative definitions of layout type may be applied, e.g. card-based layout, grid-based layout, etc. In this scenario, the context of use could be specified in the mandatory and/or preferred keys.

The specified layout type may provide a generic dashboard template, such as a generic multidisciplinary tumor board dashboard. To select more specific dashboards, a mandatory key could specify e.g. the disease conditions, i.e., to be able to select a disease-specific dashboard, the key specifying the disease condition is mandatory, e.g. (suspected) lung cancer. The mandatory key could be text- or code-based.

Preferred keys may be used to further refine the selection of the layout, e.g., by addition of extra information on the disease condition (e.g., the subtype of lung cancer) or combination of disease conditions (lung and cardiovascular disease), clinical role(s) using the dashboard (e.g., the multidisciplinary team or specific roles such as the radiologist, surgeon, etc.), the clinical context (e.g., phase in the care journey), or clinical setting (e.g., at the emergency department).

A matching score may be assigned to each of the plurality of dashboard templates. Said matching score is indicative of a relevance of the associated template information of the respective dashboard template to the dashboard request. Then, the dashboard templates are ranked according to the associated matching scores. The dashboard template is selected from the plurality of dashboard templates based on the ranking. For example, a dashboard template matches the dashboard request only if the "type" and a value of each "mandatory" key matches the provided context in the dashboard request.

Before returning the layout to the user, any adaptive layout elements may be converted to a static layout. The user receives a final static layout of dynamic form components that is ready for display

Figs. 6A and 6B illustrate the step of mapping the subgroups of clinical information categories to a dashboard template. In particular, Fig. 6A illustrates an exemplary pruned knowledge tree that has a hierarchy, and Fig. 6B illustrates an adaptive dashboard generated by mapping the subgroups of clinical information categories to the selected dashboard template. By way of example, the exemplary pruned knowledge tree shown in Fig. 6A may be of the type lung Multidisciplinary Tumor board (Lung-MDT), which may comprise a plurality of interrelated nodes representing clinical information categories to be presented. As an example, the Lung-MDT comprises a node that represents medical history, which in turn comprises two nodes representing a timeline of historical information and highlighted data from that period. The node representing highlighted data from that period may further comprise nodes representing the categories of information oncologic history and other medical history (i.e. comorbidities). Additionally, the exemplary pruned knowledge tree may have additional clinical information categories presented in an extended view, which is indicated by "... ". Such knowledge tree describes how the data elements represented by UI elements and components are related to each other. For example, Fig. 6B illustrates a dashboard that comprises hierarchal composition of a layout that can be represented by the knowledge tree shown in Fig. 6A. This insight leads to a connected hierarchy of knowledge categories and the possibility to translate this to a layout containing various UI components that are linked to the nodes and leaves of the knowledge tree.

Depending on the number of clinical information categories, the total available screen space, and the size of the UI components, the UI components may or may not fit the available area. This becomes even more challenging when these UI components have dimensions that do not neatly fit together as a puzzle. Therefore, in some implementations multiple alternative representations may be provided for the same UI component. For example, Figs. 7A-7C shown different component variants to represent various levels of details about the same medical aspect. Fig. 7A shows a summarized view. Fig. 7B shows another component variant to represent a graphic representation of the medical effect with more details. In addition to the summarized information provided by the example shown in Fig. 7A, the component variant shown in Fig. 7B further comprises a graphic representation for the organ with the abnormality, e.g., lungs in this case. In Fig. 7C, the component variant shows further details. In particular, this component variant shows a graphic representation of the lungs (left), regional lymph node stations (middle) and findings in distant organs (right). It uses circular charts to represent the diagnostic results, in which each section represents the result from a diagnostic modality. Colors may be used to indicate a level of suspicion for malignancy. With multiple alternative representations for the same UI component, the layout mechanism has the possibility to choose the combination of components that leads to the optimal layout for the knowledge to be presented according to both the properties of the knowledge categories and available screen space.

In order to generate the adaptive dashboard, both at the local level and at the global level (i.e., the entire dashboard), the system needs to decide which variants of the UI components to be displayed and in which direction they shall be "placed" onto the dashboard (e.g. take more horizontal or more vertical dashboard space). The local level may comprise a part of the layout, e.g. a dedicated card in the layout displaying a specific knowledge category. Ultimately, decisions at local levels may result in a layout that might not fit the screen size, e.g., when all selected components have a long horizontal shape and are placed in the horizontal direction. This may make calculating the optimal layout of the dashboard a constrained option-selection problem. Therefore, in some implementations, step 240, i.e., step d) may further comprise iteratively mapping the subgroup of clinical information categories to the dashboard layout elements of the selected dashboard template including resolving spatial constrains based on the priorities associated with the clinical information categories in the subgroup. As an example, in step 240, a "Tabu Search" heuristic may be implemented to find an optimal layout solution for the identified layout constraints for its performance and simplicity.

Fig. 8 illustrates a flowchart describing a method for iteratively mapping the subgroup of clinical information categories to the dashboard layout elements of the selected dashboard template.

At block 242, i.e., step d1), the step 240 may comprise generating an initial adaptive dashboard by mapping the subgroup of clinical information categories to the dashboard layout elements of the selected dashboard template. For every node in the knowledge category tree, the data processing apparatus 10 shown in Fig. 3 may select either a horizontal or vertical layout. For every leaf of the knowledge category tree, the layout can choose between the alternative UI components representing the clinical information categories in the dashboard. A solution to the layout problem may include a set of such decisions that matches the knowledge category structure and properties.

At block 244, i.e., step d2), the step 240 may further comprise determining a numeric score of the initial adaptive dashboard based on one or more of: (i) a degree of an occupied area matching the associated priority of the represented clinical information category, (ii) a proportion of empty space in the generated adaptive dashboard, and (iii) a degree of a total dashboard area size fitting an available screen area.

In other words, the "fit" for each solution may be expressed as a numeric score based on the following constraints:
How well the occupied area matches the relative priority of the represented knowledge category: e.g., a closer fit leads to a better score.
How efficient the sum of the child dashboard areas covers the enclosing area of their parent: e.g. less empty space leads to a better score.
If the total layout fits the available screen area: more overflow in either direction leads to a worse score.

At block 246, i.e., step d3), the step 240 may further comprise determining whether the determined numeric score meets pre-defined acceptance criteria. For example, the related numeric scores may be calculated and summed up, after calculation of the dashboard area sizes for all knowledge categories bottom-up using the UI components and layout directions of the proposed solution.

In response to determining that the determined numeric score does not meet the pre-defined acceptance criteria, at block 248, i.e., step d4), the step 240 may comprise the step of updating the selected dashboard template by selecting alternative dashboard layout elements representing the subgroup of clinical information categories and/or changing an layout direction of the selected dashboard template, and repeating steps d1)-d3) until the determined numeric score meets the pre-defined acceptance criteria.

In response to determining that the numeric score meets a pre-defined acceptance criteria, at block 250, i.e., step d5), the step 250 may further comprise mapping the subgroups of clinical information categories to the dashboard layout elements of the updated dashboard template.

In the exemplary method illustrated in Fig. 8, searching for the "best" layout from the many combinations of alternative components and directions of the layout on every nesting level is implemented by a constraint resolver. A constraint resolver may be an implementation of a heuristic to search for the "best" solution in a large solution space. After the "best" solution is found by solving the constraints, the actual layout is derived. This may be achieved by a top-down decomposition of the layout based on the priorities of the knowledge categories and the selected layout direction per level of the layout. This is for example needed when the best solution needs fine-tuning to use up all the available space or when some UI components overflow and need to shrink.

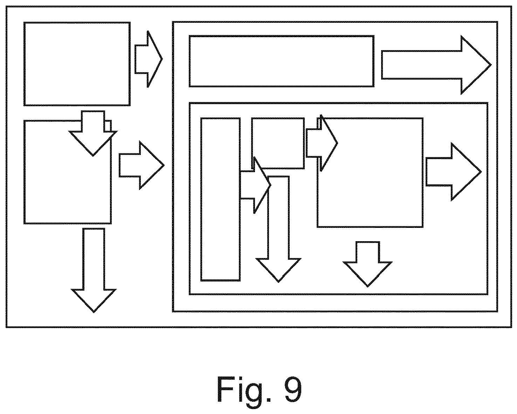

As an example, when the selected fixed-size components and layout directions are "fitting", there is a fair chance that space is left around the components when building them up according to the priorities of the knowledge categories. Fig. 9 illustrates how the remaining space can be filled by growing the selected components within the dimensions of the enclosing area.

This may be achieved by using a layout problem instance to hold the problem statement, and instances of layout solution to codify permutations of the layout options. The layout choice per knowledge category is captured in a layout value. The layout problem provides an initial layout solution to hand to the constraint resolver. Neighboring layout solutions are produced by permutating each layout value of a solution to its neighbors. The layout solution delegates calculation of its score to the layout problem, where the layout implications of the layout values of a solution compared with the knowledge category tree and the available space for the resulting layout.

Before calculating scores, the component selections and layout directions of the solution are used to calculate the implicit dimensions of every category from the bottom up. This leads to the minimal absolute dashboard area dimensions to represent each knowledge category. These dimensions are then used to calculate a set of scores that are summed to an overall score for a given solution.

The oversize score may be calculated by adding the area of any excess width and any excess height of the total layout. The area fit score may be calculated as the difference between area claimed by the knowledge category based on its priority and the actually assigned area. If the difference is negative (e.g., the assigned area is larger than necessary), the score is clipped to zero. The area efficiency score may be calculated as the difference between the area assigned to the clinical information category and the sum of the areas assigned to the children of the knowledge area. The score of a leaf category is always zero, because it is defined by the size of the assigned component variant. The overall "fit" score is calculated by summing the oversize score with the area fit scores and area efficiency scores of each category.

From this overall fit score, the optimal layout solution can be determined. The layout of this optimal layout solution, containing both the fixed and filled-in adaptive dashboard layout elements, is sent back to the original dashboard request to be rendered on the screen-based interface.

At the end, the user will be presented with the optimal layout at each decision moment, flexibly displaying the relevant subset of information from the knowledge tree, driven by availability of the data and its relative priority.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. In general, the term "or" as used herein shall only be interpreted as indicating exclusive alternatives (i.e. "one or the other but not both") when preceded by terms of exclusivity, such as "either," or "one of".

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "having," "containing," and the like are to be understood to be open-ended, i.e., to mean including but not limited to.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method for generating an adaptive dashboard for clinical decision support, the method comprising:
a) selecting (210) a knowledge tree constructed according to a set of clinical knowledge data associated with a defined disease condition and/or a defined subject context, wherein the knowledge tree comprises a plurality of interrelated nodes representing clinical information categories, the nodes having a respectively associated pruning condition;
b) determining (240) availability of the clinical information categories of the selected knowledge tree in a subject record and relevance of the clinical information categories of the selected knowledge tree to the subject context and/or the disease condition;
c) pruning (230) at least one of the nodes on the selected knowledge tree based on at least one of the pruning conditions being satisfied by the determined information availability and the determined information relevance, resulting in a pruned knowledge tree with a subgroup of clinical information categories; and
d) generating (240) the adaptive dashboard by mapping the subgroup of clinical information categories to a dashboard template.

2. The method according to claim 1, further comprising:
- receiving a dashboard request; and
- selecting the dashboard template from a plurality of dashboard templates based on the received dashboard request.

3. The method according to claim 2,
wherein the plurality of dashboard templates comprises at least one disease-specific dashboard templates being constructed for a particular disease condition.

4. The method according to claim 2 or 3,
wherein the step of selecting the dashboard template further comprises:
- assigning a score to each of the plurality of dashboard templates, said matching score indicative of a relevance of the associated template information of the respective dashboard template to the dashboard request;
- ranking the dashboard templates according to the associated matching scores; and
- selecting the dashboard template from the plurality of dashboard templates based on the ranking.

5. The method according to any one of the preceding claims,
wherein the nodes further comprise respectively associated priorities, which are usable to determine respectively associated areas in the adaptive dashboard occupied by the subgroup of clinical information categories.

6. The method according to claim 5,
wherein at least one of the respectively associated priorities is adjustable at different decision moments along a subject's clinical pathway.

7. The method according to claim 5 or 6,
wherein step d) further comprises iteratively mapping the subgroup of clinical information categories to dashboard layout elements of the selected dashboard template including resolving spatial constrains based on the priorities associated with the clinical information categories in the subgroup.

8. The method according to claim 7,
wherein the step of iteratively mapping the subgroup of clinical information categories to the dashboard layout elements of the selected dashboard template comprises:
d1) generating (242) an initial adaptive dashboard by mapping the subgroup of clinical information categories to the dashboard layout elements of the selected dashboard template;
d2) determining (244) a numeric score of the initial adaptive dashboard based on one or more of:
(i) a degree of an occupied area matching the associated priority of the represented clinical information category;
(ii) a proportion of empty space in the generated adaptive dashboard; and
(iii) a degree of a total dashboard area size fitting an available screen area;
d3) determining (246) whether the determined numeric score meets pre-defined acceptance criteria;
d4) in response to determining that the determined numeric score does not meet the pre-defined acceptance criteria, updating (248) the selected dashboard template by selecting alternative dashboard layout elements representing the subgroup of clinical information categories and/or changing an layout direction of the selected dashboard template, and repeating steps d1)-d3) until the determined numeric score meets the pre-defined acceptance criteria; and
d5) in response to determining that the numeric score meets a pre-defined acceptance criteria, mapping (250) the subgroup of clinical information categories to the dashboard layout elements of the updated dashboard template.

9. The method according to any one of the preceding claims,
wherein the clinical information categories comprise one or more of:
- subject data describing at least one physiological parameter or characteristic of the subject;
- clinical data describing at least one medical parameter or characteristic of the subject; and
- operational data describing at least one clinical test or procedure undertaken by the subject; and
- result data describing at least one result or outcome from diagnostic tests and treatment procedures.

10. The method according to any one of claims 2 to 9,
wherein the information in the dashboard request comprises one or more of:
- a disease code that specifies one disease condition or a combination of disease conditions of the subject;
- subject identifier information of the subject usable to retrieve subject-specific information, from which the disease condition and/or the subject context are derivable; and
- subject context information that specifies the subject context.

11. The method according to any one of the preceding claims,
wherein the subject context comprises one or more of:
- a position of the subject in a subject's clinical pathway;
- a clinical discipline triggering the dashboard request; and
- a context of use of the adaptive dashboard.

12. The method according to any one of the preceding claims,
wherein at least one dashboard template comprises one or more adaptive elements usable for adaptively representing different data for different subject contexts and/or different disease conditions.

13. A data processing apparatus (10) for generating an adaptive dashboard for clinical decision support, the data processing apparatus comprising a processing unit (14) configured to perform the method of any one of the preceding claims.

14. A clinical decision support system (100), comprising:
- a dashboard template database (20) configured to store a plurality of dashboard templates, each dashboard template in the plurality having a hierarchal composition of dashboard layout elements usable for presenting clinical information from one or more clinical information categories;
- a knowledge tree database (30) configured to store a plurality of knowledge trees stored in a knowledge tree database, each stored knowledge tree being constructed according to a set of clinical knowledge data associated with a defined disease condition and/or a defined subject context, wherein each knowledge tree comprises a plurality of interrelated nodes representing clinical information categories to be presented, the nodes having a respectively associated pruning condition;
- a subject case management system (40) configured to store subject records of one or more subjects; and
- a data processing apparatus (10) according to claim 13 configured to receive a dashboard request and to generate an adaptive dashboard for clinical decision support based on the received dashboard request.

15. A computer program product comprising instructions which, when the program is executed by a computer, cause the computer to carry out the method of any one of claims 1-12 and/or be configured to run on the clinical decision support system of claim 14.
